(19) Europäisches Patentamt
European Patent Office
Office européen des brevets

(11) Numéro de publication : **0 379 500 B1**

(12) # FASCICULE DE BREVET EUROPEEN

(45) Date de publication du fascicule du brevet : **18.03.92 Bulletin 92/12**

(51) Int. Cl.$^5$ : **A61K 9/50,** A61K 7/48

(21) Numéro de dépôt : **88907330.0**

(22) Date de dépôt : **11.08.88**

(86) Numéro de dépôt international : **PCT/FR88/00412**

(87) Numéro de publication internationale : **WO 89/01327 23.02.89 Gazette 89/05**

(54) **COMPOSITION DERMATOLOGIQUE AUX LIPOSOMES CONTENANT DE LA PREGNENOLONE.**

(30) Priorité : **20.08.87 FR 8711773**

(43) Date de publication de la demande : **01.08.90 Bulletin 90/31**

(45) Mention de la délivrance du brevet : **18.03.92 Bulletin 92/12**

(84) Etats contractants désignés : **AT BE CH DE FR GB IT LI LU NL SE**

(56) Documents cités :
**EP-A- 0 087 993**
**EP-A- 0 120 722**
**EP-A- 0 170 642**
**EP-A- 0 229 561**
**FR-A- 1 159 166**
**FR-A- 2 344 290**

(73) Titulaire : **PARFUMS CHRISTIAN DIOR**
**33, Avenue Hoche**
**F-75008 Paris (FR)**

(72) Inventeur : **PERRIER, Pierre**
**22, rue Jousselin**
**F-45000 Orléans (FR)**
Inventeur : **REDZINIAK, Gérard 102, rue des Fauvettes**
**Lotissement "La Petite Mairie"**
**F-45590 S.-Cyr-en-Val (FR)**
Inventeur : **ANDRE, Patrice**
**9, rue Charles-Vappereau**
**F-45170 Neuville-aux-Bois (FR)**

(74) Mandataire : **Portal, Gérard et al**
**Cabinet Beau de Loménie 55, rue d'Amsterdam**
**F-75008 Paris (FR)**

EP 0 379 500 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne essentiellement une composition à base de phases lamellaires lipidiques hydratées ou de liposomes contenant de la prégnénolone ou un ester de prégnénolone.

L'invention trouve notamment application dans le domaine cosmétique ou pharmaceutique, notamment dermatologique, pour la réalisation de compositions à activité régénératrice ou revitalisante.

Les stéroïdes ont déjà été utilisés pour la préparation de compositions pharmaceutiques ou cosmétiques destinées au traitement de la peau. Les brevets US 2 791 534 et GB 768 129 , par exemple, révèlent l'utilisation des stéroïdes comme agents à activité antiride.

L'effet des hormones sur la peau a été plus particulièrement étudié et, dans l'article publié dans J. Soc. Cosmetic Chemists, volume 18, pages 549 à 562 (19 août 1967), on indique que la testostérone produit un effet de rajeunissement sur la peau, et que la progestérone et la prégnénolone, intermédiaires dans la biosynthèse de la testostérone, produisent un effet semblable, mais à un degré bien moindre, et particulièrement faible dans le cas de la prégnénolone.

C'est pourquoi la prégnénolone, libre ou sous forme d'ester, a souvent été utilisée, pour le traitement de la peau, en combinaison avec d'autres agents actifs.

Ainsi, le brevet US 4 474 763 décrit une composition antiride contenant de la prégnénolone et de l'élastine, qui est une protéine de la peau connue pour son activité antiride.

De même, le brevet US 3 326 901 révèle une composition à action anti-irritante et antidéshydratante contenant de la prégnénolone et de l'allantoïne.

L'effet antidéshydratant sur la peau de la prégnénolone a été également mentionné plus récemment dans la demande de brevet français n° 2 405 069. Dans cette demande, il est proposé une composition pharmaceutique hormonale contenant un oestrogène hydrosoluble (oestrone), un oestrogène liposoluble (oestradiol), un androgène (la testostérone) et, éventuellement, de la prégnénolone pour donner un effet antidéshydratant à la composition. Selon l'auteur de cette demande de brevet, seules les hormones sexuelles, hormones mâles et hormones femelles, sont capables de prévenir les phénomènes de détérioration de la peau dus à l'action conjointe de l'âge et des agressions climatiques et, par contre, il est indiqué que la prégnénolone ne joue aucun rôle dans les processus de restauration cutanée.

Le brevet FR-A-1 159 166 décrit un produit cosmétique pour les soins de la peau contenant la prégnénolone et ses dérivés associés à d'autres substances pour renforcer l'activité de la prégnénolone dans le but de provoquer sur une peau veillissante un aspect tendu rajeunissant et une élimination des rides.

L'analyse de l'art antérieur montre donc que l'utilisation de la prégnénolone ou d'un ester de prégnénolone seul, dans le traitement de la peau, a toujours été limitée en raison de la très faible activité de cette substance.

Par ailleurs, on connaît déjà l'emploi de phases lamellaires lipidiques hydratées ou de liposomes dans des compositions pharmaceutiques ou des compositions cosmétiques, dans lesquelles on incorpore divers principes actifs (FR-A-2 521 565).

Il a maintenant été découvert, de manière tout à fait surprenante et inattendue, que la prégnénolone ou ses esters avaient, sur la peau et les phanères, une activité régénératrice et revitalisante exacerbée, lorsque cette substance était au moins en partie incorporée dans une phase lamellaire lipidique hydratée ou dans des liposomes. On a en particulier constaté une amélioration tout à fait remarquable de l'activité de la prègnénolone sur la synthèse des protéines.

On peut ainsi en déduire en quelque sorte un effet de potentialisation de l'activité de la prégnénolone ou des esters de la prégnénolone dans des phases lamellaires lipidiques hydratées ou dans des liposomes.

Ainsi, la présente invention a pour effet de résoudre le nouveau problème technique consistant en la fourniture d'une nouvelle formulation de la prégnénolone ou d'un ester de prégnénolone, permettant de potentialiser leur efficacité pour permettre leur utilisation dans des compositions cosmétiques ou pharmaceutiques, notamment dermatologiques, à activité régénératrice ou revitalisante.

Ainsi, selon un premier aspect, la présente invention fournit une composition à base de phases lamellaires lipidiques hydratées ou de liposomes, caractérisée en ce que lesdites phases lamellaires lipidiques hydratées ou lesdits liposomes contiennent au moins en partie de la prégnénolone ou un ester de prégnénolone, de préférence l'acétate, le sulfate ou le palmitate de prégnénolone.

Selon un autre mode de réalisation avantageux de l'invention, la composition précitée est caractérisée en ce que les phases lamellaires lipidiques hydratées précitées ou les liposomes contiennent en outre au moins en partie un stérol, de préférence le β-sitostérol.

Plus particulièrement, le stérol est incorporé dans la phase lipidique des phases lamellaires lipidiques hydratées ou des liposomes à une concentration en poids comprise entre 1 % et 20 %, et de préférence égale à 10 % environ.

Selon une variante de réalisation de cette composition, celle-ci est caractérisée en ce que la prégnénolone ou l'ester de prégnénolone, seul ou en mélange avec le stérol, est introduit dans la phase lipidique des phases lamellaires lipidiques hydratées ou des liposomes.

Plus particulièrement, la concentration en prégnénolone ou en ester de prégnénolone est comprise entre 0,05 % et 20 %, de préférence entre 1 % et 15 %, et de préférence encore entre 1 % et 5 % en poids de ladite phase lipidique, de sorte que la somme des concentrations dans cette phase en prégnénolone ou en ester de prégnénolone d'une part et en stérol, s'il y est présent, d'autre part ne dépasse pas environ 25 % et de préférence environ 20 %.

Selon une autre variante de réalisation de cette composition, celle-ci est caractérisée en ce que l'ester de prégnénolone est introduit dans la phase aqueuse des phases lamellaires lipidiques hydratées ou des liposomes. Dans ce cas, bien entendu, la solubilité dans l'eau dudit ester devra être telle que l'on puisse obtenir au moins la concentration souhaitée dans lesdites phases lamellaires ou lesdits liposomes.

Plus particulièrement, la concentration de l'ester de prégnénolone est comprise entre 0,001 % et 5 %, et de préférence entre 0,01 % et 2 % en poids de ladite phase aqueuse.

L'incorporation de la prégnénolone ou de ses esters, et éventuellement du stérol, dans les phases lamellaires lipidiques hydratées ou dans les liposomes selon l'invention,peut être effectuée selon des procédés connus. Ceux-ci sont choisis plus particulièrement en fonction du caractère plus ou moins lipophile ou hydrophile du composé à incorporer.

Selon un mode préféré de réalisation de la composition à base de phases lamellaires lipidiques hydratées ou liposomes selon l'invention, on choisit une technique de préparation décrite dans le document FR-A-2 521 565, éventuellement en combinaison avec une technique décrite dans le document FR-A-2 534 587.

Selon un deuxième aspect, la présente invention concerne aussi une composition cosmétique ou pharmaceutique, notamment dermatologique, à activité régénératrice ou revitalisante, caractérisée en ce qu'elle comprend une composition à base de phases lamellaires lipidiques hydratées ou de liposomes, telle que précédemment définie.

De préférence, la proportion en poids de la prégnénolone ou de l'ester de prégnénolone est comprise entre 0,0005 % et 5 % , de préférence entre 0,002 % et 1 %, et encore de préférence entre 0,01 et 0,5 % en poids, relativement au poids total de la composition.

D'autres buts,caractéristiques et avantages de l'invention apparaîtront plus clairement à la lumière de la description explicative qui va suivre, faite en référence à plusieurs exemples illustratifs . Dans ces exemples, les pourcentages sont donnés en poids, sauf indication contraire.

EXEMPLE 1

A - Préparation d'une composition sous forme de poudre lipidique contenant de l'acétate de prégnénolone.

On dissout dans 100 ml de dichlorométhane 100 mg d'acétate de prégnénolone, 1 g de β-sitostérol auxquels on ajoute 8,9 g de lécithine de soja, éventuellement en présence d'un antioxydant lipophile, par exemple 0,2 g d'α-tocophénol.

On atomise la solution obtenue à 65°C, comme décrit dans le document FR-A-2 521 565. On obtient ainsi une poudre lipidique composée de petites particules sensiblement sphériques de 1 à 40 nm de diamètre environ.

B - Préparation d'une composition sous forme de suspension de liposomes avantageusement homogénéisée.

On disperse 5 g de la poudre obtenue à l'étape A dans une quantité d'eau telle que l'on aboutisse à un volume final de 250 ml. Cette opération est conduite à température ordinaire sous agitation magnétique pendant 1 h.

On obtient ainsi, après homogénéisation soit au moyen d'ultrasons, soit au moyen d'un homogénéiseur sous pression, par exemple selon le procédé décrit dans le document FR-A-2 534 487, une suspension de liposomes.

Si, par exemple, on réalise une homogénéisa:ion par traitement aux ultrasons pendant 15 min, on obtient des liposomes ayant une dimension comprise entre 135 et 186 nm.

On obtient donc,après cette étape B, 250 ml de suspension de liposomes homogénéisés, dont la teneur en phase lipidique est de 2 %.

EXEMPLES 2 à 7

Compositions sous forme de suspensions de liposomes contenant de l'acétate de prégnénolone.

On prépare plusieurs compositions sous forme de suspensions de liposomes selon le procédé décrit à l'exemple 1, en faisant varier la proportion des constituants de la phase lipidique.

Le tableau I ci-après indique la composition des différentes suspensions obtenues.

EXEMPLES 8 à 10

Compositions sous forme de suspensions de liposomes contenant de la prégnénolone.

On prépare, selon le procédé de l'exemple 1, différentes compositions liposomales en substituant la prégnénolone à l'acétate de prégnénolone et en faisant varier la proportion des constituants de la phase lipidique. La composition des différentes suspensions de liposomes figure au tableau I.

TABLEAU I

| Exemple n° | | 1 | 2 | 3 | 4 | 5 | 6 | 7 | 8 | 9 | 10 |
|---|---|---|---|---|---|---|---|---|---|---|---|
| Composition de la poudre lipidique, % en poids | lécithine de soja | 89 | 80 | 85 | 80 | 75 | 70 | 90 | 89 | 80 | 80 |
| | β-sitostérol | 10 | 10 | 10 | 10 | 15 | 20 | 0 | 10 | 10 | 10 |
| | acétate de prégné-nolone | 1 | 10 | 5 | 10 | 10 | 10 | 10 | 0 | 0 | 0 |
| | prégnénolone | 0 | 0 | 0 | 0 | 0 | 0 | 0 | 1 | 10 | 10 |
| Concentrations dans la sus-pension de liposomes, % en poids | phase lipidique | 2 | 20 | 2 | 2 | 2 | 2 | 2 | 2 | 10 | 30 |
| | acétate de prégné-nolone | 0,02 | 2 | 0,1 | 0,2 | 0,2 | 0,2 | 0,2 | 0 | 0 | 0 |
| | prégnénolone | 0 | 0 | 0 | 0 | 0 | 0 | 0 | 0,02 | 1 | 3 |

EXEMPLE 11

Etude de l'influence du stérol sur la stabilité des liposomes contenant de la prégnénolone ou un ester de prégnénolone.

On cherche à déterminer l'influence du β-sitostérol sur la stabilité des liposomes selon l'invention.

Pour cela, on prépare une suspension témoin selon le procédé de l'exemple 1, dont la phase lipidique a comme composition 90 % de lécithine de soja et 10 % de β-sitostérol.

L'évaluation de la stabilité des liposomes est effectuée par la détermination de leur taille au moyen d'un appareil approprié, tel qu'un NANOSIZER®. Les mesures sont effectuées au temps zéro (à l'achèvement de la préparation), à température ambiante, puis à 8 j et à 15 j aux températures de 4°C, ambiante, et 40°C. Le tableau II mentionne, pour chaque mesure, la polydispersité qui quantifie l'éparpillement des tailles des liposomes et la taille moyenne. Plus la valeur de ces grandeurs est faible et régulière dans le temps, généralement meilleure est la qualité de la suspension de liposomes, dont la stabilité en est un critère.

La stabilité des liposomes peut donc être évaluée par une mesure de leur taille, car on sait que lorsqu'ils sont instables les liposomes ont tendance à fusionner entre eux, leur dimension augmente et, à partir d'une certaine taille, ils sédimentent.

Le tableau II montre clairement que l'incorporation de l'acétate de prégnénolone est favorisée par la présence de β-sitostérol. Lorsque la teneur en β-sitostérol dans la poudre est nulle (ex. n° 7), on observe une sédimentation au bout de 8 j à 40°C, de plus, les valeurs de la polydispersité et de la taille, sont grandes.

Dans les autres cas, la taille des liposomes reste sensiblement constante et à une valeur acceptable.

En outre, le tableau II montre que l'incorporation de β-sitostérol à un taux supérieur à environ 15 % en poids (ex. n° 6), dans la poudre lipidique, provoque des phénomènes de sédimentation et nuit à la stabilité des liposomes contenant l'acétate de prégnénolone.

On voit clairement l'avantage de la présence de β-sitostérol, dont la concentration est de préférence 10 % en poids de la phase lipidique (ex. n° 4).

TABLEAU II

| Exemple n° | | | 3 | 4 | 5 | 6 | 7 | Témoin |
|---|---|---|---|---|---|---|---|---|
| Composition de la poudre lipidique, % en poids | lécithine | | 85 | 80 | 75 | 70 | 90 | 90 |
| | β-sitostérol | | 10 | 10 | 15 | 20 | 0 | 10 |
| | acétate de prégnénolone | | 5 | 10 | 10 | 10 | 10 | 0 |
| Taille des liposomes (polydispersité/taille (nm)) | t = 0 | T.A. | 6/272 | 6/260 | 4/268 | 3/284 | 9/1200 | 3/140 |
| | t = 8 j | 4°C | 5/247 | 5/252 | 4/294 | 4/290 | 9/1040 | 5/134 |
| | | T.A. | 7/386 | 5/253 | 5/292 | 4/300 | 9/1560 | 3/152 |
| | | 40°C | 5/233 | 4/242 | 3/300 | 4/305 +sédiment. | sédiment. | 3/178 |
| | t = 15 j | 4°C | | 7/420 | 4/299 | 4/310 | | |
| | | T.A. | | 6/300 | 4/320 | 4/294 +sédiment. | | |
| | | 40°C | | 4/275 +sédiment. | 4/277 +sédiment. | 4/353 +sédiment. | | |

t = temps en jours

T.A. = température ambiante (environ 20°C)

EXEMPLE 12

Evaluation du pouvoir multiplicateur des compositions selon l'invention sur des fibroblastes humains en culture.

La méthode d'évaluation du pouvoir multiplicateur de substances sur des cellules en culture est dérivée de la méthode de Madame ADOLPHE et al., J. Cosmetic Sci. (1983), 6, 55.

Des fibroblastes humains sont cultivés en boîte de Petri (35 mm) dans du MEM (milieu minimum de Eagle) contenant 5 % de sérum de veau.

Après 24 h (37°C, 5 % $CO_2$), on remplace le milieu par un mélange constitué de MEM et de la substance à tester, à différentes concentrations. Ce mélange contient une concentration de 2,5 % en sérum de veau. Un témoin à 2,5 % de sérum de veau, sans substance additionnelle, est réalisé en parallèle.

Les cellules sont ensuite incubées à 37°C en atmosphère humide chargée de 5 % de $CO_2$ pendant 7 j. Trois essais sont effectués pour chaque culture.

Le dénombrement des cellules est réalisé au moyen d'un appareillage approprié, après lavage au tampon phosphate et décollement des cellules à la trypsine.

Le pouvoir multiplicateur des substances est exprimé par la formule suivante :

$$A.P. = \frac{N.P. - N\ 2{,}5}{N\ 2{,}5} \times 100$$

- dans laquelle A.P. représente le pouvoir multiplicateur des cellules, N.P. le nombre des cellules cultivées en présence de la substance à tester et N 2,5 le nombre de cellules cultivées dans le témoin, c'est-à-dire en présence de 2,5 % de sérum de veau.

Les résultats obtenus pour différentes concentrations en acétate de prégnénolone, en abrégé PREAC, sont représentés à la figure 1 sous forme d'un histogramme.

La figure 1 révèle que l'acétate de prégnénolone, libre ou incorporé en liposomes ne présente aucun effet significatif sur l'augmentation du nombre des fibroblastes humains en culture.

EXEMPLE 13

Evaluation de l'activité stimulante sur la synthèse des protéines.

L'évaluation de l'activité sur le taux de protéine est réalisée suivant une méthode qui découle de la méthode décrite par JOHNSON et J.A. LOTT (1978), Clin. Chem. 24.

A la fin de la culture réalisée précédemment à l'exemple 12, les boites sont lavées deux fois au tampon phosphate puis traitées avec 1 ml de soude (0,5 N).

Après agitation, 30 µl de surnageant cellulaire sont ajoutés à 2 ml d'une solution de bleu de Coomassie.

La concentration en protéines est obtenue après lecture au proti-analyser (Marius Instruments) qui évalue automatiquement le rapport des densités optiques.

$$\frac{D0\ 590\ nm}{D0\ 465\ nm}$$

L'activité en "taux de protéine" pour chaque substance, à chaque concentration est exprimée par la formule :

$$T.P. = \frac{T_S - T_{2,5}}{T_{2,5}} \times 100$$

- dans laquelle T.P. représente l'activité en taux de protéine ; $T_S$ représente le taux de protéine obtenu sur des cultures en présence du produit à tester et $T_{2,5}$ représente le taux de , protéine obtenu sur des cultures en présence du témoin, c'est-à-dire en présence de 2,5 % de sérum de veau.

On a représenté à la figure 2 l'histogramme des résultats de l'activité sur le taux de protéine à différentes concentrations en acétate de prégnénolone.

La figure 2 met très nettement en évidence l'action stimulante de l'acétate de prégnénolone incorporé en liposomes sur la synthèse des protéines, alors que ce même produit, à l'état libre, ne montre pratiquement aucune action.

En conclusion, les tests effectués sur les cultures de fibroblastes humains montrent l'intérêt des compositions selon l'invention pour l'entretien, ou la restauration, des processus biologiques normaux de la peau et des phanères, par le biais de l'action stimulante sur la synthèse des protéines tissulaires.

En effet, on sait d'une part que,lorsque les cellules se divisent, cette synthèse est ralentie. L'absence d'action particulière des compositions selon l'invention sur la division des cellules ne constitue donc pas un handicap. D'autre part, l'on connaît l'importance que présente l'existence des protéines tissulaires en quantité et en qualité, pour le bon état physiologique de la peau. L'action stimulante des compositions selon l'invention sur la synthèse de telles protéines constitue donc un très grand intérêt, notamment en cosmétique et en dermatologie.

EXEMPLE 14

Composition gélifiée à base de liposomes

| | |
|---|---|
| lécithine de soja | 0,89 g |
| β-sitostérol | 0,10 g |
| acétate de prégnénolone | 0,01 g |
| excipient gélifié qsp | 100 g |

On prépare tout d'abord une poudre lipidique selon l'exemple 1-A. Cette poudre est ensuite dispersée à la concentration de 4 % selon l'exemple 1-B dans de l'eau, éventuellement stabilisée au moyen d'un conservateur,cosmétiquement ou pharmaceutiquement acceptable à la concentration habituelle.

On gélifie alors la suspension obtenue en la mélangeant, à raison d'une partie pour 3 parties, avec un gel aqueux de Carbopol® 940, préparé séparément de façon classique à la concentration de 5 %.

La composition gélifiée obtenue peut être utilisée soit pour être incorporée dans des formulations cosmétiques ou pharmaceutiques, soit telle quelle, éventuellement additionnée de parfums, pour le soin de la peau du corps ou du visage, avantageusement en applications quotidiennes.

EXEMPLE 15

Composition cosmétique sous forme de crème

| | |
|---|---|
| lécithine de soja | 4 g |
| β-sitostérol | 0,5 g |
| acétate de prégnénolone | 0,5 g |
| excipient pour émulsion huile dans eau qsp | 100 g |

On prépare séparément une suspension de liposomes selon l'exemple 2 d'une part, et une émulsion huile dans eau d'autre part. Ces deux préparations sont ensuite mélangées à raison de 1 volume de suspension liposomale pour 3 volumes d'émulsion.

La crème obtenue est appliquée de préférence le soir sur le visage.

EXEMPLE 16

Composition dermatologique cicatrisante

| | |
|---|---|
| lécithine de soja | 8 g |
| β-sitostérol | 1 g |
| acétate de prégnénolone | 1 g |
| excipients gélifiés qsp | 100 g |

Cette composition est obtenue à partir de la poudre préparée selon l'exemple 4 et dispersée à la concentration de 20 % au moyen d'un homogénéiseur sous pression. La dispersion obtenue est ensuite mélangée avec 1 volume équivalent d'un gel de Carbopol® 940 à 5 %.

Cette composition présente d'excellentes propriétés cicatrisantes. Elle est indiquée, en particulier, pour prévenir et traiter les vergetures en applications biquotidiennes.

## Revendications

1. Composition à base de phases lamellaires lipidiques hydratées ou de liposomes, caractérisée en ce que lesdites phases lamellaires lipidiques hydratées ou lesdits liposomes contiennent au moins en partie de la prégnénolone ou un ester de prégnénolone tel que notamment l'acétate, le sulfate ou le palmitate de prégnénolone.

2. Composition selon la revendication 1, caractérisée en ce que les phases lamellaires lipidiques hydratées précitées ou les liposomes contiennent en outre au moins en partie un stérol, et de préférence un ß-sitostérol.

3. Composition selon la revendication 2, caractérisée en ce que le stérol est incorporé dans la phase lipidique des phases lamellaires lipidiques hydratées ou des liposomes à une concentration en poids comprise entre 1 % et 20 %, et de préférence égale à 10 % environ.

4. Composition selon l'une quelconque des revendications 1 à 3, caractérisée en ce que la prégnénolone ou l'ester de prégnénolone précité est introduit dans la phase lipidique des phases lamellaires lipidiques hydratées ou des liposomes.

5. Composition selon la revendication 4, caractérisée en ce que la concentration en prégnénolone ou en ester de prégnénolone est comprise entre 0,05 et 20 %, et de préférence entre 1 % et 15 %, et de préférence encore entre 1 % et 5 % en poids de ladite phase lipidique, et de sorte que la somme des concentrations dans cette phase en prégnénolone ou en ester de prégnénolone d'une part et en stérol, s'il y est présent, d'autre part ne dépasse pas environ 25 % et de préférence environ 20 %.

6. Composition selon l'une des revendications 1 à 3, caractérisée en ce que l'ester de prégnénolone précité est introduit dans la phase aqueuse des phases lamellaires lipidiques hydratées ou des liposomes.

7. Composition selon la revendication 6, caractérisée en ce que la concentration de l'ester de prégnénolone est comprise entre 0,001 % et 5 %, et de préférence entre 0,01 % et 2 % en poids de ladite phase aqueuse.

8. Composition cosmétique ou pharmaceutique, notamment dermatologique, à activité régénératrice ou revitalisante, caractérisée en ce qu'elle comprend une composition à base de phases lamellaires lipidiques hydratées ou de liposomes contenant au moins en partie de la prégnénolone ou un ester de prégnénolone tel que notamment l'acétate, le sulfate ou le palmitate de prégnénolone.

9. Composition cosmétique ou pharmaceutique, notamment dermatologique, selon la revendication 8, caractérisée en ce que les phases lamellaires lipidiques hydratées précitées ou les liposomes contiennent en outre un stérol, notamment le β-sitostérol.

10. Composition cosmétique ou pharmaceutique, notamment dermatologique, selon la revendication 9, caractérisée en ce que le stérol est incorporé dans la phase lipidique des phases lamellaires lipidiques hydratées ou des liposomes à une concentration en poids comprise entre 1 % et 20 %, et de préférence égale à 10 % environ.

11. Composition cosmétique ou pharmaceutique, notamment dermatologique, selon l'une des revendications 8 à 10, caractérisée en ce que la prégnénolone ou l'ester de prégnénolone précité est introduit dans la phase lipidique des phases lamellaires lipidiques hydratées ou des liposomes.

12. Composition cosmétique ou pharmaceutique, notamment dermatologique, selon la revendication 11, caractérisée en ce que la concentration en prégnénolone ou en ester de prégnénolone est comprise entre 0,05 et 20 % et de préférence entre 1 % et 15 %, et de préférence encore entre 1 % et 5 % en poids de ladite phase lipidique, et de sorte que la somme des concentrations dans cette phase en prégnénolone ou en ester de prégnénolone d'une part et en stérol, s'il y est présent, d'autre part ne dépasse pas environ 25 % et de préférence environ 20 %.

13. Composition cosmétique ou pharmaceutique, notamment dermatologique, selon l'une des revendications 8 à 10, caractérisée en ce que l'ester de prégnénolone est introduit dans la phase aqueuse des phases lamellaires lipidiques hydratées ou des liposomes.

14. Composition cosmétique ou pharmaceutique, notamment dermatologique, selon la revendication 13, caractérisée en ce que la concentration de l'ester de prégnénolone est comprise entre 0,001 % et 5 %, et de préférence entre 0,01 % et 2 % en poids de ladite phase aqueuse.

15. Composition cosmétique ou pharmaceutique, notamment dermatologique, selon l'une des revendications 8 à 14, caractérisée en ce que la proportion en poids de prégnénolone au d'ester de prégnénolone est comprise entre 0,0005 % et 5 %, de préférence entre 0,002 et 1 %, et encore de préférence entre 0,01 et 0,5 % en poids par rapport au poids total de la composition.

16. Composition cosmétique ou pharmaceutique, notamment dermatologique, à activité de stimulation sur la synthèse des protéines tissulaires, caractéristée en ce qu'elle comprend une composition à base de phases lamellaires lipidiques hydratées ou de liposomes contenant au moins en partie de la prégnénolone ou un ester de prégnénolone tel que notamment l'acétate, le sulfate ou le palmitate de prégnénolone, en particulier tel que défini selon l'une quelconque des revendications 1 à 7.

17. Composition dermatologique à activité cicatrisante, caractérisée en ce qu'elle comprend une composition à base de phases lamellaires lipidiques hydratées ou de liposomes contenant au moins en partie de la prégnénolone ou un ester de prégnénolone tel que notamment l'acétate, le sulfate ou le palmitate de prégnénolone, en particulier tel que défini selon l'une quelconque des revendications 1 à 7.

18. Utilisation d'une composition à base de phases lamellaires lipidiques hydratées ou de liposomes telle que définie à l'une quelconque des revendications 1 à 7, pour la fabrication d'une composition cosmétique ou pharmaceutique, notamment dermatologique, à activité régénératrice ou revitalisante, ou cicatrisante.

## Patentansprüche

1. Zusammensetzung auf Basis von hydratisierten lamellaren Lipidphasen oder von Liposomen, dadurch gekennzeichnet, daß die hydratisierten lamellaren Lipidphasen oder die Liposomen zumindest zum Teil Pregnenolon oder einen Pregnenolonester, wie insbesondere Pregnenolonacetat, -sulfat oder -palmitat, enthalten.

2. Zusammensetzung nach Anspruch 1, dadurch gekennzeichnet, daß die hydratisierten lamellaren Lipidphasen oder die Liposomen weiters zumindest zum Teil ein Sterin, vorzugsweise β-Sitosterin, enthalten.

3. Zusammensetzung nach Anspruch 2, dadurch gekennzeichnet, daß das Sterin in der Lipidphase der hydratisierten lamellaren Lipidphasen oder der Liposomen in einer Gewichtskonzentration zwischen 1 % und 20 %, vorzugsweise von etwa 10 %, eingearbeitet ist.

4. Zusammensetzung nach einem der Ansprüche 1 bis 3, dadurch gekennzeichnet, daß das Pregnenolon oder der Pregnenolonester in die Lipidphase der hydratisierten lamellaren Lipidphasen oder der Liposomen eingebracht ist.

5. Zusammensetzung nach Anspruch 4, dadurch gekennzeichnet, daß die Konzentration an Pregnenolon oder an Pregnenolonester zwischen 0,05 und 20 Gew.%, vorzugsweise zwischen 1 und 15 Gew.% und noch bevorzugter zwischen 1 und 5 Gew.% der Lipidphase beträgt, u.zw. derart, daß die Summe der Konzentrationen an Pregnenolon oder an Pregnenolonester einerseits und gegebenenfalls an Sterin anderseits in dieser Phase etwa 25 %, vorzugsweise etwa 20 %, nicht übersteigt.

6. Zusammensetzung nach einem der Ansprüche 1 bis 3, dadurch gekennzeichnet, daß der Pregnenolonester in die wässerige Phase der hydratisierten lamellaren Lipidphasen oder der Liposomen eingebracht ist.

7. Zusammensetzung nach Anspruch 6, dadurch gekennzeichnet, daß die Konzentration an Pregnenolonester zwischen 0,001 Gew.% und 5 Gew.%, vorzugsweise zwischen 0,01 Gew.% und 2 Gew.%, der wässerigen Phase beträgt.

8. Kosmetische oder pharmazeutische, insbesondere dermatologische Zusammensetzung mit regenerierender oder revitalisierender Aktivität, dadurch gekennzeichnet, daß sie eine Zusammensetzung auf Basis von hydratisierten lamellaren Lipidphasen oder von Liposomen umfaßt, die zumindest zum Teil Pregnenolon oder einen Pregnenolonester, wie insbesondere Pregnenolonacetat, -sulfat oder -palmitat, enthalten.

9. Kosmetische oder pharmazeutische, insbesondere dermatologische Zusammensetzung nach Anspruch 8, dadurch gekennzeichnet, daß die hydratisierten lamellaren Lipidphasen oder die Liposomen weiters ein Sterin, vorzugsweise β-Sitosterin, enthalten.

10. Kosmetische oder pharmazeutische, insbesondere dermatologische Zusammensetzung nach Anspruch 9, dadurch gekennzeichnet, daß das Sterin in die Lipidphase der hydratisierten lamellaren Lipidphasen oder der Liposomen in einer Gewichtskonzentration zwischen 1 % und 20 %, vorzugsweise etwa 10 %, eingearbeitet ist.

11. Kosmetische oder pharmazeutische, insbesondere dermatologische Zusammensetzung nach einem der Ansprüche 8 bis 10, dadurch gekennzeichnet, daß das Pregnenolon oder der Pregnenolonester in die Lipidphase der hydratisierten lamellaren Lipidphasen oder der Liposomen eingebracht ist.

12. Kosmetische oder pharmazeutische, insbesondere dermatologische Zusammensetzung nach Anspruch 11, dadurch gekennzeichnet, daß die Konzentration an Pregnenolon oder an Pregnenolonester zwischen 0,05 und 20 Gew.%, vorzugsweise zwischen 1 und 15 Gew.% und noch bevorzugter zwischen 1 und 5 Gew.% der Lipidphase beträgt, u.zw. derart, daß die Summe der Konzentrationen an Pregnenolon oder an Pregnenolonester einerseits und gegebenenfalls an Sterin anderseits in dieser Phase etwa 25 %, vorzugsweise etwa 20 %, nicht übersteigt.

13. Kosmetische oder pharmazeutische, insbesondere dermatologische Zusammensetzung nach einem der Ansprüche 8 bis 10, dadurch gekennzeichnet, daß der Pregnenolonester in in die wässerige Phase der hydratisierten lamellaren Lipidphasen oder der Liposomen eingebracht ist.

14. Kosmetische oder pharmazeutische, insbesondere dermatologische Zusammensetzung nach Anspruch 13, dadurch gekennzeichnet, daß die Konzentration an Pregnenolonester zwischen 0,001 Gew.% und 5 Gew.%, vorzugsweise zwischen 0,01 Gew.% und 2 Gew.%, der wässerigen Phase beträgt.

15. Kosmetische oder pharmazeutische, insbesondere dermatologische Zusammensetzung nach einem der Ansprüche 8 bis 14, dadurch gekennzeichnet, daß das Gewichtsverhältnis von Pregnenolon oder Pregenenolonester zwischen 0,005 Gew.% und 5 Gew.%, vorzugsweise zwischen 0,002 Gew.% und 1 Gew.% und noch bevorzugter zwischen 0,01 Gew.% und 0,5 Gew.%, bezogen auf das Gesamtgewicht der Zusammensetzung, beträgt.

16. Kosmetische oder pharmazeutische, insbesondere dermatologische Zusammensetzung mit stimulierender Wirkung auf die Synthese der Gewebsproteine, dadurch gekennzeichnet, daß sie eine Zusammensetzung auf Beasis von hydratisierten lamellaren Lipidphasen oder von Liposomen umfaßt, die zumindest zum Teil Pregnenolon oder einen Pregnenolonester, wie insbesondere Pregnenolonacetat, -sulfat oder -palmitat, enthalten, insbesondere wie in einem der Ansprüche 1 bis 7 definiert.

17. Dermatologische Zusammensetzung mit narbenbildender Aktivität, dadurch gekennzeichnet, daß sie eine Zusammensetzung auf Basis von hydratisierten lamellaren Lipidphasen oder von Liposomen umfaßt, die zumindest zum Teil Pregnenolon oder einen Pregnenolonester, wie insbesondere Pregnenolonacetat, -sulfat oder -palmitat, enthalten, insbesondere wie in einem der Ansprüche 1 bis 7 definiert.

18. Verwendung einer Zusammensetzung auf Basis von hydratisierten lamellaren Lipidphasen oder von Liposomen, wie in einem der Ansprüche 1 bis 7 definiert, zur Herstellung einer kosmetischen oder pharmazeutischen, insbesondere dermatologischen Zusammensetzung mit regnerierender oder revitalisierender oder narbenbildender Aktivität.

## Claims

1. A composition based on hydrated lipidic lamellar phases or on liposomes, wherein pregnenolone or a pregnenolone ester, such as, in particular, pregnenolone acetate, sulfate or palmitate, is at least partially incorporated in the said hydrated lipidic lamellar phases or the said liposomes.

2. A composition according to claim 1, wherein a sterol, preferably ß-sitosterol, is also at least partially incorporated in the above-mentioned hydrated lipidic lamellar phases or the liposomes.

3. A composition according to claim 2, wherein the sterol is incorporated in the lipidic phase of the hydrated lipidic lamellar phases or of the liposomes at a concentration by weight of between 1% and 20% and preferably of about 10%.

4. A composition according to any one of claims 1 to 3, wherein the above-mentioned pregnenolone or pregnenolone ester is introduced into the lipidic phase of the hydrated lipidic lamellar phases or of the liposomes.

5. A composition according to claim 4, wherein the concentration of pregnenolone or pregnenolone ester is between 0.05 and 20%, preferably between 1% and 15% and more preferably between 1% and 5% of the weight of the said lipidic phase, so that the sum of the concentrations in this phase of pregnenolone or pregnenolone ester, on the one hand, and any sterol present, on the other, does not exceed about 25% and preferably about 20%.

6. A composition according to any one of claims 1 to 3, wherein the above-mentioned pregnenolone ester is introduced into the aqueous phase of the hydrated lipidic lamellar phases or of the liposomes.

7. A composition according to claim 6, wherein the concentration of the pregnenolone ester is between 0.001% and 5% and preferably between 0.01% and 2% of the weight of the said aqueous phase.

8. A cosmetic or pharmaceutical composition, notably a dermatological composition, with regenerating or revitalizing activity, which comprises a composition based on hydrated lipidic lamellar phases or on liposomes in which pregnenolone or a pregnenolone ester, such as, notably, pregnenolone acetate, sulfate or palmitate, is at least partially incorporated.

9. A cosmetic or pharmaceutical composition, notably a dermatological composition, according to claim 8, wherein the above-mentioned hydrated lipidic lamellar phases or the liposomes also contain a sterol, notably β-sitosterol.

10. A cosmetic or pharmaceutical composition, notably a dermatological composition, according to claim 9, wherein the sterol is incorporated in the lipidic phase of the hydrated lipidic lamellar phases or of the liposomes at a concentration by weight of between 1% and 20% and preferably of about 10%.

11. A cosmetic or pharmaceutical composition, notably a dermatological composition, according to any one of claims 8 to 10, wherein the above-mentioned pregnenolone or pregnenolone ester is introduced into the lipidic phase of the hydrated lipidic lamellar phases or of the liposomes.

12. A cosmetic or pharmaceutical composition, notably a dermatological composition, according to claim 11, wherein the concentration of pregnenolone or pregnenolone ester is between 0.05 and 20%, preferably between 1% and 15% and more preferably between 1% and 5% of the weight of the said lipidic phase, so that the sum of the concentrations in this phase of pregnenolone or pregnenolone ester, on the one hand, and any sterol present, on the other, does not exceed about 25% and preferably about 20%.

13. A cosmetic or pharmaceutical composition, notably a dermatological composition, according to any one of claims 8 to 10, wherein the pregnenolone ester is introduced into the aqueous phase of the hydrated lipidic lamellar phases or of the liposomes.

14. A cosmetic or pharmaceutical composition, notably a dermatological composition, according to claim 13, wherein the concentration of the pregnenolone ester is between 0.001% and 5% and preferably between 0.01% and 2% of the weight of the said aqueous phase.

15. A cosmetic or pharmaceutical composition, notably a dermatological composition, according to any one of claims 8 to 14, wherein the proportion by weight of pregnenolone or pregnenolone ester is between 0.0005% and 5%, preferably between 0.002 and 1% and more preferably between 0.01 and 0.5% by weight, relative to the total weight of the composition.

16. A cosmetic or pharmaceutical composition, notably a dermatological composition, having a stimulating activity on the synthesis of tissular proteins, wherein it comprises a composition based on hydrated lipidic lamellar phases or on liposomes, containing at least in part pregnenolone or a pregnenolone ester such as notably a pregnenolone acetate, sulfate or palmitate, in particular as defined according to any one of claims 1 to 7.

17. A dermatological composition having a cicatrizing activity, wherein it comprises a composition based on hydrated lipidic lamellar phases or on lyposomes containing at least in part pregnenolone or a pregnenolone ester such as notably pregnenolone acetate, sulfate or palmitate, in particular as defined according to any one of claims 1 to 7.

18. Use of a composition based on hydrated lipidic lamellar phases or on liposomes such as defined according to any one of claims 1 to 7, for the manufacture of a cosmetic or pharmaceutical composition, notably a dermatological composition, having a regenerating or revitalizing or cicatrizing activity.

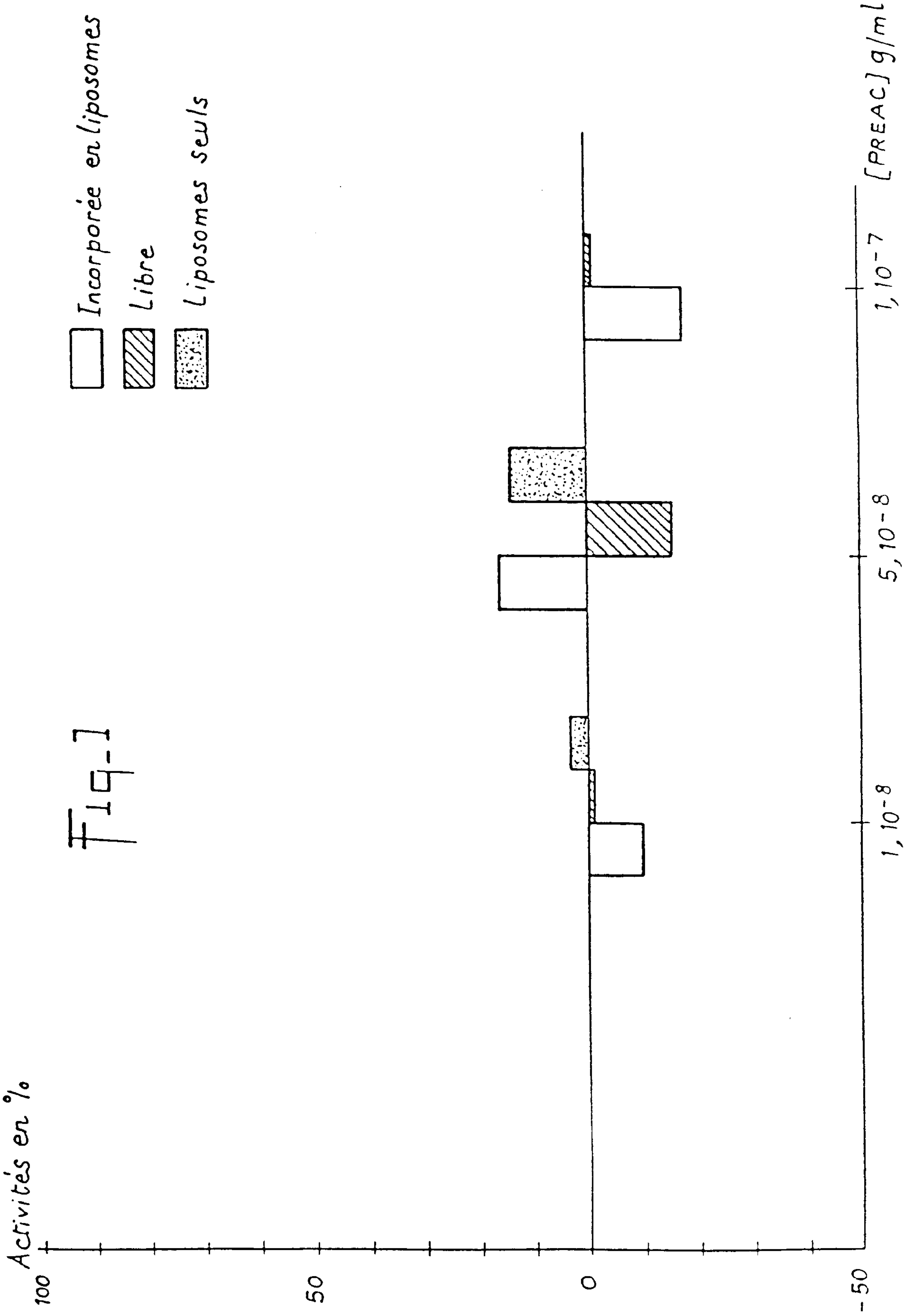
Fig-1
Activités en %
100
50
0
-50
Incorporée en liposomes
Libre
Liposomes seuls
1,10-8
5,10-8
1,10-7
[PREAC] g/ml

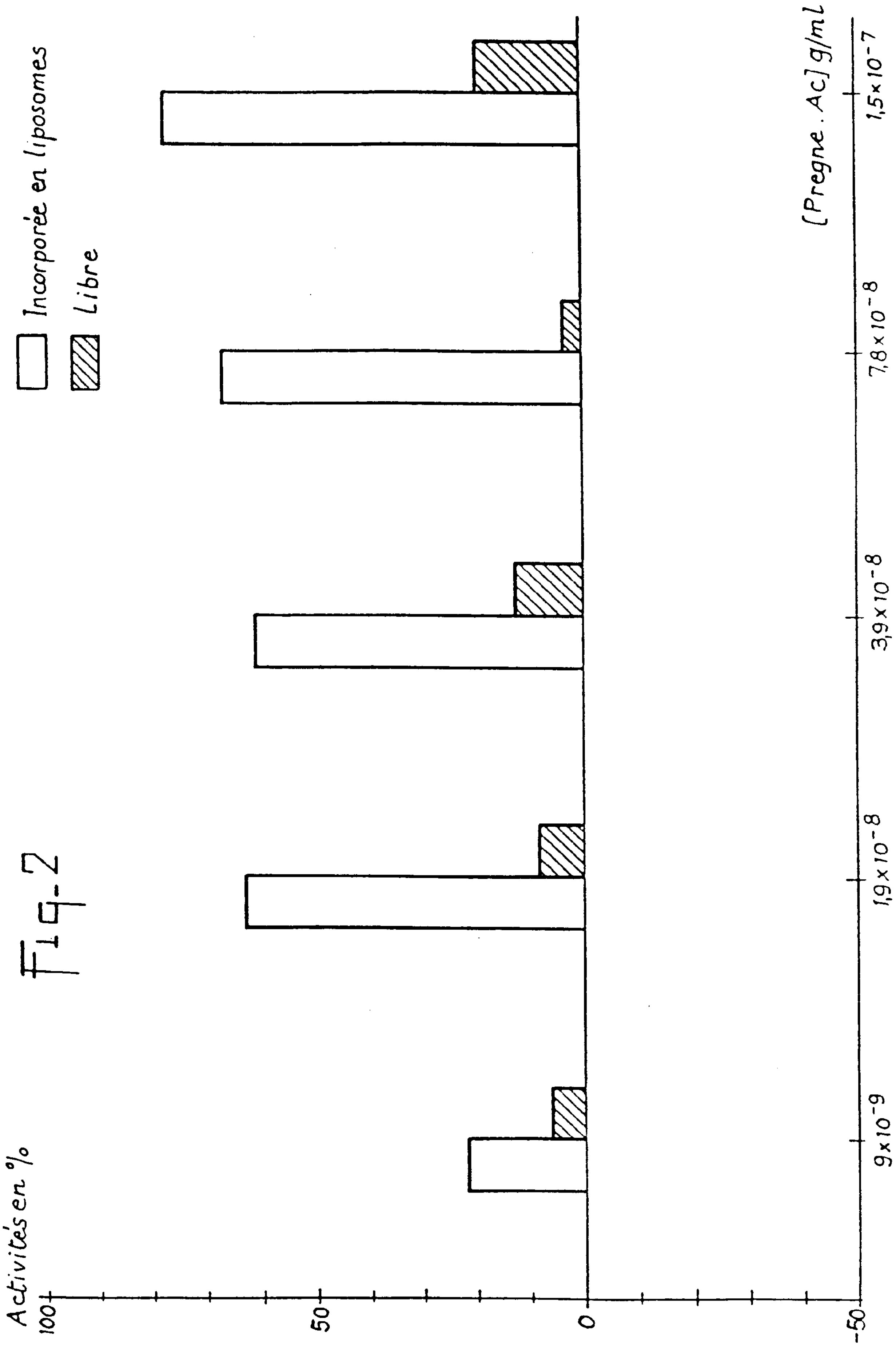
Activités en %
100
50
0
-50
Incorporée en liposomes
Libre
[Pregne. Ac] g/ml
9x10⁻⁹
1,9x10⁻⁸
3,9x10⁻⁸
7,8x10⁻⁸
1,5x10⁻⁷

Fig-2